# EUROPÄISCHE PATENTSCHRIFT

(11) **EP 0 791 072 B1**
(45) Veröffentlichungstag und Bekanntmachung des Hinweises auf die Patenterteilung: **17.05.2000**
(21) Anmeldenummer: 95938413.2
(22) Anmeldetag: 06.11.1995
(51) Int. Cl.: C12Q 1/42, C12Q 1/48

(54) **VERFAHREN ZUR BESTIMMUNG DER AKTIVITÄT SPEZIFISCHER PHOSPHOTYROSINPHOSPHATASEN UND SPEZIFISCHER EFFEKTOREN DAVON IN INTAKTEN ZELLEN**
PROCESS FOR DETERMINING THE ACTIVITY OF SPECIFIC PHOSPHOTYROSINE PHOSPHATASES AND SPECIFIC EFFECTORS THEREOF IN INTACT CELLS
PROCEDE DE DETERMINATION DE L'ACTIVITE DE PHOSPHOTYROSINE PHOSPHATASES SPECIFIQUES ET D'EFFECTEURS SPECIFIQUES DE CELLES-CI DANS DES CELLULES INTACTES

(30) Priorität: 07.11.1994 DE 4439662
(43) Veröffentlichungstag der Anmeldung: 27.08.1997
(73) Patentinhaber: Max-Planck-Gesellschaft zur Förderung der Wissenschaften e.V. Berlin, 80539 München (DE)
(72) Erfinder: ULLRICH, Axel, D-80798 München (DE); BÖHMER, Sylvia-Annette, D-07747 Jena (DE); BÖHMER, Frank, D-07747 Jena (DE); OBERMEIER, Axel, D-93049 Regensburg (DE)
(74) Vertreter: Weiss, Wolfgang, Dipl.-Chem. Dr.
(86) Internationale Anmeldenummer: EP9504342
(87) Internationale Veröffentlichungsnummer: WO9614433

(56) Entgegenhaltungen:
- EP-A- 0 165 430
- EP-A- 0 294 538
- WO-A-95/23231
- DE-A- 3 704 389
- CHEMICAL ABSTRACTS, Band 120, Nr. 1, veröffentlicht 1994, 3 Januar, (Columbus, Ohio, USA), J. KREMENSKOTHEN et al. "Determination of phosphotyrosine phosphatase (PTPase) activity in normal and transformed cells", Seite 585, Nr. 5194x; & Mol. Cell. Biochem. 1993, 125(1), 1-9.
- CHEMICAL ABSTRACTS, Band 115, Nr. 21, veröffentlicht 1991, 25 November, (Columbus, Ohio, USA), J. BABCOOK et al. "Automated nonisotopic assay for protein-tyrosine kinase and protein-tyrosine phosphatase activities", Seite 415, Nr. 226 642r; & Anal. Biochem. 1991, 196(2), 245-51.
- BIOCHEMICAL AND BIOPHYSICAL RESEARCH COMMUNICATIONS, Band 107, Nr. 3, veröffentlicht 1982, 16 August, G. SWARUP et al. "Inhibition of membrane phosphotyrosyl-protein phosphatase activity by vanadate" Seiten 1104-1109,

## Beschreibung

Die vorliegende Erfindung betrifft ein Verfahren zur Aktivitätsbestimmung von Proteintyrosinphosphatasen und zur Identifizierung von Effektoren davon.

Die Phosphorylierung von zellulären Proteinen an Tyrosinresten ist ein allgemeines Grundprinzip zur Regulation bzw. Steuerung von zellulären Vorgängen und Zuständen. Von besonderer Bedeutung ist dieser Mechanismus beispielsweise bei der Signalübertragung in Zellen sowie bei der Regulation von Zellproliferation und -differenzierung (Ullrich und Schlessinger (1990)). Proteine, die als Antwort auf ein bestimmtes Signal spezifisch phosphoryliert werden, umfassen sowohl Proteine an der Zelloberfläche, wie etwa Rezeptoren für Wachstumsfaktoren, Hormone etc., wie auch intrazelluläre Proteine.

Die "Einstellung" des Phosphorylierungsspiegels erfolgt durch Proteintyrosinkinasen (PTK) und ihre erst in den letzten Jahren genauer charakterisierten Gegenspieler, die Proteintyrosinphosphatasen (PTPasen) (Charbonneau und Tonks (1992); Pot und Dixon (1992)).

Aufgrund der Bedeutung der Proteinphosphorylierung an Tyrosinresten bei Zellproliferation und -differenzierung wird diesen beiden Enzymklassen ein großes Interesse entgegengebracht. Die Bestimmung der Aktivität spezifischer PTK und PTPasen in vivo erlaubt z.B. wertvolle Rückschlüsse auf die Beteiligung der Proteintyrosinphosphorylierung an pathologischen Proliferationsprozessen, und natürliche und synthetische Effektoren beider Enzymklassen sind Wirkstoffe mit hoher potentieller medizinischer Relevanz für die Beeinflussung derartiger Vorgänge.

Übliche, aus dem Stand der Technik bekannte Verfahren zur Bestimmung von Enzymaktivitäten oder zur Identifizierung von Effektoren davon umfassen die Identifizierung und Isolierung des jeweiligen Enzyms und in vitro Untersuchungen. Die Identifizierung spezifischer PTPasen, beispielsweise der PTPasen für den PDGF-Rezeptor oder den NGF-Rezeptor, ist jedoch schwierig und bisher nicht gelungen. Deshalb kann gegenwärtig kein Testsystem für diese PTPasen aufgebaut werden, das auf isolierten Enzymen beruht. Weiterhin wird die Spezifität der PTPasen offenbar in hohem Maße durch die räumliche Nachbarschaft zu den zugeordneten Substraten in der Zelle bedingt. Die Isolation der PTPasen führt zumindest partiell zum Verlust derartiger Spezifitäten und somit würden Untersuchungen an isolierten PTPasen möglicherweise ein falsches Bild liefern. In analoger Weise ist auch die Spezifität von Effektoren häufig im zellulären Kontext anders als in isolierten Systemen.

Weiterhin sind aus dem Stand der Technik Verfahren bekannt, die es erlauben, die Aktivität spezifischer PTPasen in situ zu bestimmen, wobei diese Verfahren die Möglichkeit voraussetzen, das jeweilige Substrat für die spezifischen PTK und PTPasen zu isolieren und den Phosphotyrosingehalt dieses Substrats zu analysieren. Derartige Verfahren umfassen bei Verwendung von ³²P die Zugabe von nicht-radioaktivem ATP zum Ansatz und die anschließende Verfolgung der Phosphorylierung/Dephosphorylierung des Substrats (Swarup et al. (1982); Böhmer et al. (1993); Faure et al. (1992)) oder die Hemmung der PTK durch Chelatoren, wie etwa EDTA, der für die Kinasereaktion essentiellen divalenten Kationen und Verfolgung des Phosphotyrosingehalts. Ein Nachteil dieser bekannten Systeme besteht in ihrer geringen Spezifität, da bei ihnen alle Tyrosinkinasen in einer Testprobe ohne Unterschied getroffen werden, wodurch Rückschlüsse auf eine spezifische Wirkung unmöglich gemacht werden. Ein weiterer wesentlicher Nachteil ist auch darin zu sehen, daß weder Chelatoren noch ATP rasch in Zellen permeieren können, so daß die Anwendung dieser Verfahren somit die vorherige Zellpermeabilisierung oder Lyse der Zellen voraussetzt (Mooney und Bordwell (1992)). Infolgedessen sind diese Verfahren ungeeignet, die in vivo Aktivität von spezifischen PTPasen in einer intakten Zelle korrekt zu bestimmen.

In EP 0 165 430 A1 werden pharmazeutische Zubereitungen beschrieben, die ein Ca²⁺-Ionophor wie z.B. A23187 enthalten können und die die Wundgranulation und Epithelisation fördern. Im genannten Dokument wird offenbart, daß A23187 in Kombination mit Calcium in einer Konzentration von 3x10⁻⁸ bis 3x10⁻⁶ M wirkt, wobei die Ca²⁺-Ionen im extrazellulären Raum nur in einer Konzentration von 0,1 bis 50 mM vorhanden sein müssen, um einen ausreichenden Ca²⁺-Zufluß in die Zelle sicherzustellen. Ca²⁺-Ionophore werden in diesem Dokument definiert als Verbindungen, die die Plasmamembran selektiv für Ca²⁺-Ionen permeabilisieren.

EP 0 294 538 A2 offenbart ein Mittel zur Behandlung von Viruserkrankungen, das als Wirkstoff Nigerizin enthält. Nigerizin wird im genannten Dokument als ionophores Antibiotikum bezeichnet, das insbesondere gegen DNA-enthaltende Viren, wie z.B. Herpesvirus, Adenovirus und Pockenvirus wirkt. Nigerizin wird z.B. von Streptomyces hygroscopicus produziert.

Ein Hinweis auf Verfahren zur Messung von PTPase-Aktivitäten findet sich nicht.

Es ist daher die Aufgabe der vorliegenden Erfindung, ein Verfahren zur Messung spezifischer PTPase-Aktivitäten bereitzustellen, das die aus dem Stand der Technik bekannten Nachteile mindestens teilweise vermeidet und das an einer intakten Zelle durchführbar ist.

Diese Aufgabe wird gelöst durch ein Verfahren zur Bestimmung der Aktivität einer spezifischen Proteintyrosinphosphatase (PTPase) in intakten Zellen, welches dadurch gekennzeichnet ist, daß man
(a) intakte Zellen, in denen ein Substrat der zu bestimmenden PTPase enthalten ist, gegebenenfalls nach Stimulierung der Substratphosphorylierung, mit mindestens einer Substanz, welche in die intakte Zelle eindringen und selektiv die Phosphorylierung des Substrats inhibieren kann, in einer wirksamen Konzentration inkubiert,
(b) nach einer gegebenen Inkubationsdauer den Phosphorylierungsgrad des Substrats mißt,
(c) gegebenenfalls Schritt (b) mindestens einmal wiederholt und
(d) aus dem gemessenen Phosphorylierungsgrad des Substrats die Aktivität der spezifischen PTPase bestimmt.

Die Inhibierung der Substratphosphorylierung durch die Substanz kann durch unterschiedliche Wirkungsweisen hervorgerufen werden, wie etwa durch Wechselwirkung mit der Phosphorylierungsstelle des Substrats oder durch Wechselwirkung mit einer spezifischen Proteintyrosinkinase. In einer besonderen Ausführungsform ist die Substanz ein spezifischer PTK-Inhibitor. Im folgenden wird die Wirkung dieser Substanz zumeist einfach als Inhibition der spezifischen PTK bezeichnet. Es ist jedoch beabsichtigt, daß andere Wirkungsweisen ebenfalls von der vorliegenden Erfindung umfaßt werden.

Durch die Hemmung der PTK wird in vivo die fortlaufende Phosphorylierung der spezifischen Substrate unterbunden und auf diese Weise die Wirkung der diese Substrate dephosphorylierenden spezifischen PTPasen sichtbar gemacht. Ein wesentlicher Vorteil der erfindungsgemäßen Verfahren besteht darin, daß die Substratphosphorylierung selektiv inhibiert wird, wodurch Nebenwirkungen, die bei Verwendung der bisher üblichen unselektiven Hemmstoffe unvermeidlich waren, auf ein Minimum reduziert werden. Ein weiterer Vorteil des erfindungsgemäßen Verfahrens gegenüber den aus dem Stand der Technik bekannten Systemen besteht darin, daß die spezifischen PTK-Inhibitoren rasch in intakte Zellen permeieren können. Eine Zellpermeabilisierung ist dabei nicht erforderlich und somit wird die in vivo Situation mit Ausnahme der spezifisch blockierten PTK intakt belassen.

Zur Durchführung des erfindungsgemäßen Verfahrens werden Zellen, die das Substrat exprimieren, für einen vorgegebenen Zeitraum mit mindestens einem spezifischen PTK-Inhibitor inkubiert. Danach werden die Zellen vorzugsweise aufgeschlossen und der Phosphorylierungsgrad des Substrats bestimmt. Zur Bestimmung der in vivo Aktivität von PTPasen werden vorzugsweise mindestens zwei Messungen nach unterschiedlichen Inkubationszeiträumen durchgeführt, um den Zeitverlauf der Substratdephosphorylierung zu ermitteln. Um eine Verfälschung des Meßergebnisses zu vermeiden, ist es zuvor normalerweise notwendig, das Substrat von anderen störenden Bestandteilen abzutrennen. Verfahren dazu sind dem Fachmann bekannt und umfassen z.B. Immunpräzipitation und dgl.. Die Bestimmung des Phosphorylierungsgrads des Substrats kann durch in der Technik bekannte Verfahren, beispielsweise durch Verwendung von Anti-Phosphotyrosin-Antikörpern erfolgen.

Als Zellen eignen sich im Prinzip alle prokaryontischen und eukaryontischen Zellen, die das Substrat exprimieren, wobei aufgrund der medizinischen Relevanz Säugerzellen und insbesondere Humanzellen bevorzugt sind. Aus Gründen der einfacheren Handhabbarkeit werden im allgemeinen herkömmliche Zellinien, wie etwa Swiss 3T3- oder PC12-Zellen, verwendet.

Zur Verbesserung der Meßgenauigkeit ist es im allgemeinen bevorzugt, sicherzustellen, daß das Substrat für die getestete PTPase in einer im wesentlichen phosphorylierten Form vorliegt. In den Fällen, bei denen das Substrat erst infolge eines bestimmten Signals phosphoryliert wird, ist es üblicherweise bevorzugt, die Wirkung dieses Signals auszulösen. Beispielsweise kann bei Oberflächenrezeptoren, die erst nach Bindung eines Liganden durch die spezifischen PTK phosphoryliert werden, vor Zugabe des PTK-Inhibitors eine Inkubation mit dem jeweiligen Liganden durchgeführt werden. In anderen Fällen kann es auch bevorzugt sein, eine Präinkubation mit Inhibitor, gefolgt von Stimulierung durch Liganden durchzuführen, insbesondere wenn ein lediglich qualitatives Ergebnis durch Vergleich mit einer Kontrollbestimmung in Abwesenheit von Inhibitor angestrebt wird. In nochmals anderen Fällen kann es bevorzugt sein, keine Stimulation durchzuführen, z.B. wenn eine geeignete Phosphorylierung des Substrats bereits durch einen bestimmten Typ der verwendeten Zelle gegeben ist.

Als selektiver PTK-Inhibitor kann jede Substanz in einer wirksamen Konzentration verwendet werden, die die Phosphorylierung des Substrats bzw. eine spezifische PTK des Substrats selektiv hemmt und die in intakte Zellen eindringen kann. Unter einer wirksamen Konzentration wird diejenige Konzentration verstanden, bei der eine mindestens weitgehende Inaktivierung der spezifischen Substratphosphorylierung erreicht wird und gleichzeitig keine Nebenwirkungen beobachtet werden, die das Meßergebnis beeinträchtigen oder/und für die Zellen toxisch sind. Im allgemeinen werden Konzentrationen im Bereich von etwa 0,1 bis 100 µM verwendet. Die Auswahl der Inhibitorsubstanz erfolgt auf Basis des Substrats bzw. der zu inhibierenden PTK.

PTK-Inhibitoren sind aus dem Stand der Technik bekannt (Levitzky (1992); Burke (1992)). Die in der Beschreibung erwähnten bzw. in den Beispielen der vorliegenden Erfindung verwendeten Substanzen umfassen z.B. die Chinoxalinderivate AG1295, AG1296 sowie das Staurosporinderivat K252a mit den folgenden Strukturformeln:

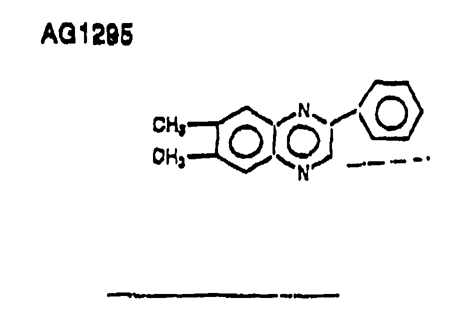

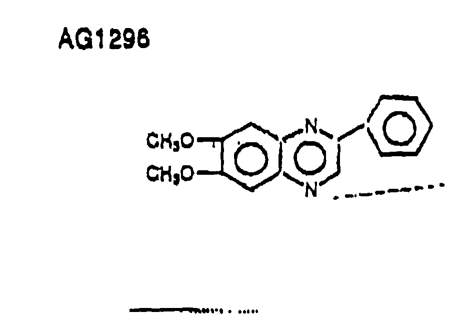

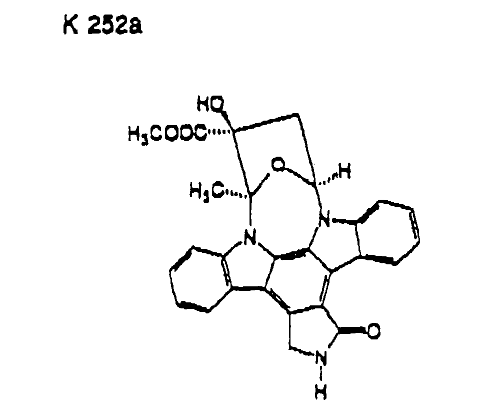

Die spezifische PTK, die bei Durchführung des erfindungsgemäßen Verfahrens selektiv gehemmt wird, stellt nicht notwendigerweise ein eigenständiges Enzym dar. Die PTK kann beispielsweise ein mit dem Substrat assoziiertes Protein sein oder auch eine Untereinheit des Substrats, so daß die Phosphorylierung eine Autophosphorylierung ist.

Das Substrat der zu bestimmenden PTPase kann ein beliebiges zelluläres Protein sein und es ist lediglich wichtig, daß dieses Protein von spezifischen PTK phosphoryliert und von spezifischen PTPasen dephosphoryliert wird. Derartige Substrate umfassen beispielsweise intrazelluläre Proteine, wie Kernproteine, oder membranassoziierte Proteine, ebenso wie membranständige Proteine z.B. an der Zelloberfläche. Es wurde festgestellt, daß als Substrat für das erfindungsgemäße Verfahren Zelloberflächenrezeptoren, wie z.B. Rezeptoren für einen Wachstumsfaktor, etwa NGF-Rezeptor, PDGF-Rezeptor, KIT/SCF-Rezeptor, KD-Rezeptor, EGF-Rezeptor und Insulinrezeptor, hervorragend geeignet sind.

Eine geeignete Kombination von Protein und PTK-Inhibitor umfaßt beispielsweise die Verwendung von PDGF-Rezeptor als Substrat und PTK-Inhibitor AG 1295 und/oder AG 1296. Diese Kombination eignet sich somit zum Bestimmen der Aktivität von PTPasen, die auf den PDGF-Rezeptor wirken bzw. zum Auffinden von Effektoren dieser PTPasen. Eine andere bevorzugte Kombination umfaßt einen NGF-Rezeptor und K252a als PTK-Inhibitor, wodurch die Aktivität der auf einen NGF-Rezeptor wirkenden PTPasen bzw. Effektoren davon ermittelt werden können.

In einer besonderen Ausführungsform erlaubt das Verfahren der vorliegenden Erfindung die Identifizierung von Effektoren der spezifischen PTPase. Dazu wird die Aktivität der spezifischen PTPase gemäß dem erfindungsgemäßen Verfahren bestimmt und zusätzlich eine Bestimmung in Gegenwart einer Testsubsstanz durchgeführt. Durch Vergleich der Phosphorylierungsgrade des Substrats mit und ohne Testsubstanz kann die Wirkung der Testsubstanz auf die Aktivität der spezifischen PTPase ermittelt werden.

Derartige Effektoren sind aufgrund ihrer potentiellen Fähigkeit, pathologische Proliferations- und Differenzierungsprozesse zu beeinflussen, in hohem Maße medizinisch relevant.

Es wird beispielsweise angenommen, daß PDGF eine Schlüsselrolle für unerwünschte Hyperproliferationen in bestimmten Tumoren, bei Arteriosklerose, Arthritis und Fibrosen zukommt (Heldin und Westermark (1991); Ross, (1993); Rubin et al. (1988); Shaw et al. (1991)). Aktivatoren der diesen Signalweg negativ regulierenden PDGF-Rezeptor PTPasen können als spezifische Cytostatika zur Beeinflussung dieser pathologischen Prozesse eingesetzt werden. NGF und verwandte neurotrophe Faktoren spielen eine entscheidende Rolle für die neuronale Differenzierung (Snider (1994)). Eine ungenügende Aktivität dieser differenzierungsinduzierenden Regulation ist wahrscheinlich die Grundlage verschiedener neurodegenerativer Erkrankungen (Barinaga (1994)). Inhibitoren der den NGF-Signalweg negativ regulierenden PTPasen sind potentiell neurotrophe Wirkstoffe und können zur Behandlung derartiger Erkrankungen eingesetzt werden. Eine ähnliche Situation existiert im Falle des nicht-insulinabhängigen Diabetes mellitus (NIDDM), dem eine Unterfunktion des Insulin-Rezeptors zugrunde liegt (Goldstein (1992)). Inhibitoren der auf den Insulin-Rezeptor gerichteten PTPase(n) haben eine Insulin-mimetische Funktion in Tiermodellen, jedoch sind bisher nur relativ toxische und unspezifische derartige Substanzen bekannt. Bei Verfügbarkeit spezifischer Inhibitoren für die Insulin-Rezeptor PTK kann das erfindungsgemäße Verfahren auch zur Auffindung von spezifischen Inhibitoren der Insulin-Rezeptor PTPasen und damit selektiver Insulin-Mimetika genutzt werden.

Es wurde z.B. festgestellt, daß bestimmte Ionophore die Aktivität von spezifischen PTPasen selektiv beeinflussen können, daß sie also als Effektoren wirksam sind. Derartige Ionophore sind natürliche oder synthetische Stoffe, die einen Ionentransport, wie etwa von Na⁺, K⁺, Ca²⁺ und Mg²⁺ durch Membranen bewirken. Als Ionophore sind sowohl Kanalbildner als auch mobile Carrier bekannt, es wird jedoch davon ausgegangen, daß eine selektive Wirkung im wesentlichen durch die mobilen Carrier vermittelt wird. Ein spezifisches Beispiel eines Effektors ist ein Ionophor, das zweiwertige Ionen und insbesondere Ca²⁺ und/oder Mg²⁺ transportiert, wie z.B. das Ionophor A 23187, 5-(Methylamino)-2-[[3,9,11-trimethyl-8-[1-methyl-2-oxo-(1H-pyrrol-2-yl)ethyl]-1,7-dioxaspiro[5,5]undec-2-yl]methyl]-4-benoxazolcarbonsäure 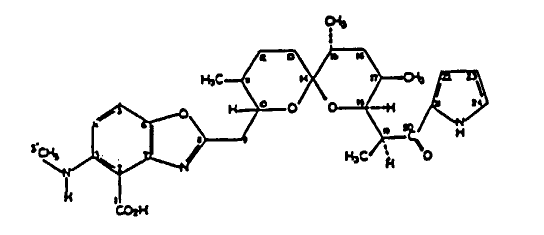 oder Derivate davon (Debono et al. (1981)). A23187 wirkt als Aktivator von PTPasen, die spezifisch PDGF-Rezeptor dephosphorylieren. Die Wirkung von A 23187 ist in Beispiel 3 gezeigt.

Das Ionophor kann als Wirkstoff einer pharmazeutischen Zusammensetzung zur Beeinflussung der Wirkung von PTPasen in intakten Zellen vorliegen, die gegebenenfalls übliche pharmazeutische Hilfs-, Verdünnungs- und Trägermittel enthält.

Der Wirkstoff ist dabei in einer Menge vorhanden, die die erwünschte Hemmung der spezifischen PTPase verursacht und im wesentlichen keine toxischen Nebenwirkungen hervorruft. Obwohl die Dosierung vom jeweiligen Wirkstoff abhängig ist, kann im allgemeinen davon ausgegangen werden, daß eine wirksame Menge etwa 0,01 bis 100 mg/kg Körpergewicht beträgt. Die pharmazeutische Zusammensetzung wird durch ein geeignetes Verfahren verabreicht. Derartige Verabreichungsformen sind dem Fachmann bekannt, wie beispielsweise orale, subkutane, intravenöse, intramuskuläre Verabreichung und dgl.. Der Wirkstoff ist in der Zusammensetzung in einer Form enthalten, die einen Transport zum Zielgewebe erlaubt. Derartige Formen umfassen in Abhängigkeit vom Wirkstoff und der Verabreichungsform beispielsweise wäßrige oder ölige Lösungen, Liposomen, Depotpräparate und dgl.

Der Wirkstoff der pharmazeutischen Zusammensetzung ist vorzugsweise ein Ionophor, das Mg²⁺ und/oder Ca²⁺ transportiert. Die pharmazeutische Zusammensetzung enthält vorzugsweise das Ca-Ionophor A23187 zur Aktivierung von gegen PDGF-Rezeptor gerichteter PTPase-Aktivität.

Die folgenden Beispiele erläutern in Verbindung mit den Abbildungen das erfindungsgemäße Verfahren weiter.
Abb. 1 zeigt die Hemmung der PDGF-Rezeptor PTK durch AG 1296,
Abb. 2 zeigt die Aktivität der PDGF-Rezeptor-PTPasen bei Verwendung von AG 1296,
Abb. 3 zeigt eine Ausführungsform des erfindungsgemäßen Verfahrens in Swiss 3T3 Zellen,
Abb. 4 zeigt eine graphische Auswertung des Versuchs von Abb. 3,
Abb. 5 zeigt die Hemmung der NGF-Rezeptor PTK durch K252a,
Abb. 6 zeigt eine andere Ausführungsform des erfindungsgemäßen Verfahrens in PC12-Zellen.

### Beispiel 1

### Messung der PDGF-Rezeptor-gerichteten PTPase(n) in Swiss 3T3 Zellen mit Hilfe eines spezifischen Inhibitors der PDGF-Rezeptor-PTK

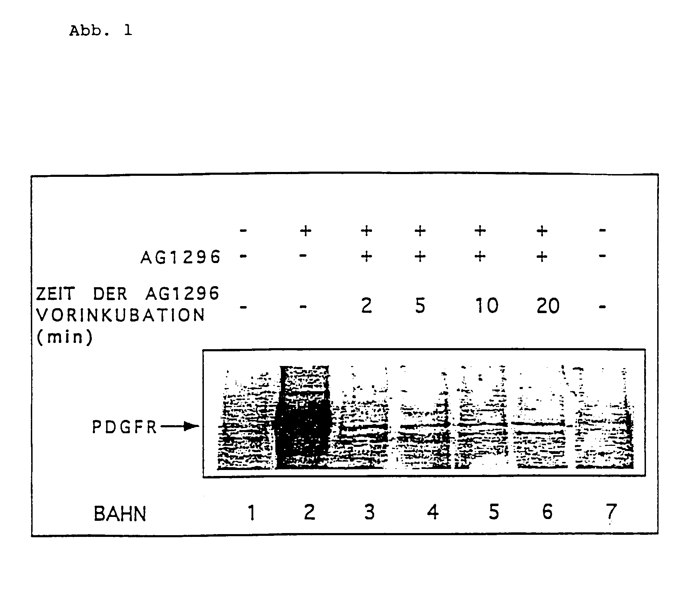

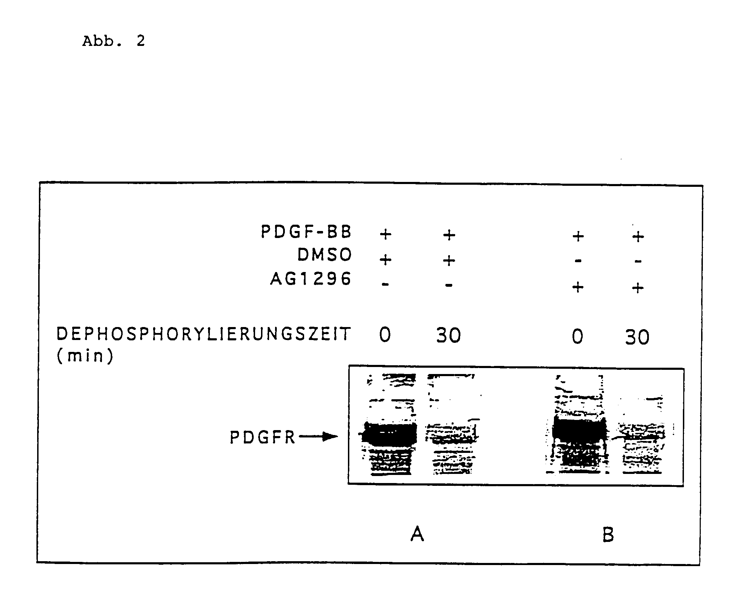

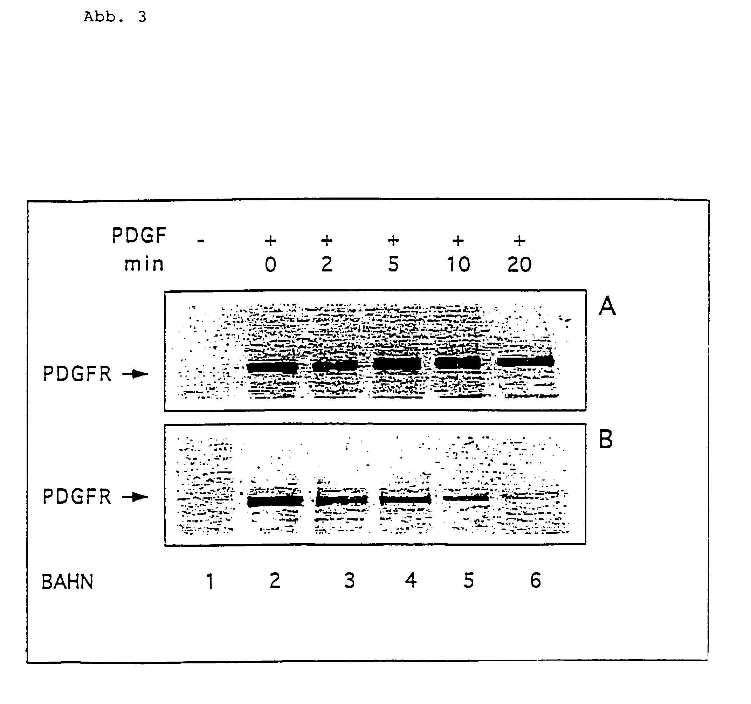

Swiss 3T3 Fibroblasten (Maus) exprimieren PDGFα und PDGFβ-Rezeptoren auf der Zelloberfläche (Hosang et al. (1989)). Die Stimulation der Zellen mit PDGF führt zu einer raschen Autophosphorylierung dieser Rezeptoren an Tyrosinresten. Diese Autophosphorylierung ist in Immunoblots mit Anti-Phosphotyrosin-Antikörpern nach Immunpräzipitation der Rezeptoren und in unfraktionierten Zell-Lysaten nachweisbar. Die autophosphorylierten Rezeptoren werden durch PDGF-Rezeptor-gerichtete PTPase(n) rasch dephosphoryliert (Bohmer et al. (1993)). Die Identität der zellulären PTPasen, die diese Reaktion katalysieren, ist nicht bekannt. Ein hochspezifischer Inhibitor für die PDGF-Rezeptor PTK (AG 1296) hemmt deren Aktivität bereits nach sehr kurzer Behandlung der Zellen (2 min) vollständig (Abb. 1). In isolierten Membranen von Swiss 3T3-Zellen beeinflußt der Inhibitor die in den Membranen enthaltenen PTPase(n) für den autophosphorylierten PDGF-Rezeptor in ihrer Aktivität nicht (Abb. 2). Wird der Inhibitor Zellen nach Aktivierung der PDGF-Rezeptor PTK zugesetzt, kann die Dephosphorylierung des PDGF-Rezeptors zeitabhängig verfolgt werden (Abb. 3).

### Hemmung der PDGF-Rezeptor PTK durch den spezifischen Inhibitor AG 1296 (Abb. 1)

Konfluente, ruhende Swiss 3T3-Zellen wurden in serumfreiem Zellkulturmedium ohne Zusätze (Bahn 1,2) oder mit dem PDGF-Rezeptor PTK Inhibitor AG 1296 (Endkonzentration 50 µM) (Bahn 3-6) für verschiedene Zeiten, wie angegeben, inkubiert. Dann erfolgte die Zugabe von humanem rekombinantem PDGF-BB (Endkonzentration 100 ng/ml) (Bahn 2-6) oder des entsprechenden Lösungsmittels (Bahn 1) und weitere Inkubation für 5 min. Nach Standardverfahren wurde dann ein Zellextrakt hergestellt und vergleichbare Mengen des Extrakts wurden mittels SDS-PAGE und Immunoblotting mit Anti-Phosphotyrosin-Antikörpern analysiert.

Die PDGF-induzierte Autophosphorylierung des Rezeptors wird bereits durch Inkubation der Zellen für 2 min mit AG 1296 vollständig unterdrückt.

### AG 1296 hemmt nicht die PDGF-Rezeptor-gerichtete(n) PTPase(n) (Abb. 2)

Isolierte Swiss 3T3-Zellmembranen (10 µg Protein per Bahn) wurden mit PDGF (2 µg/ml) für 20 min auf Eis behandelt. Durch Zusatz von 3 mM (Endkonzentration) ATP erfolgte dann die Autophosphorylierung der aktivierten PDGF-Rezeptor PTK. Die Kinase-Reaktion wurde durch Zugabe von 10 mM EDTA gehemmt. Gleichzeitig wurde AG 1296 (50 µM) (B) oder das entsprechende Lösungsmittel (DMSO) (A) zugesetzt. Direkt nach Zusatz der Agenzien (0 min) oder nach 30 min Inkubation wurden der Phosphotyrosingehalt des PDGF-Rezeptors analog wie in Abb. 1 beschrieben gemessen. Das stark abgeschwächte Signal nach 30 min ist auf die dephosphorylierende Wirkung der PTPase(n) zurückzuführen. AG 1296 beeinflußt diese Dephosphorylierung nicht.

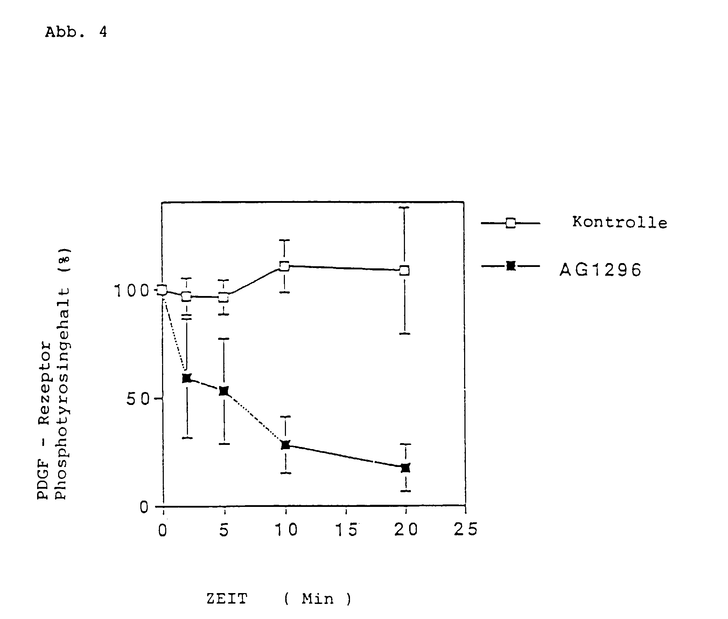

### Behandlung von intakten Swiss 3T3 Zellen mit AG 1296 erlaubt die Detektion der Aktivität PDGF-Rezeptor-gerichteter PTPase(n) in vivo (Abb. 3 und 4)

Swiss 3T3-Zellen wurden in serumfreiem Medium mit PDGF (Abb. 3, Bahn 2-6) oder dem entsprechenden Lösungsmittel (Abb. 3, Bahn 1) behandelt. Nach 5-minütiger Stimulation wurde AG 1296 (50 µM, Abb. 3 B) oder entsprechendes Lösungsmittel (DMSO, Abb. 3 A) zugesetzt. Zu verschiedenen Zeitpunkten, wie angegeben, wurden die entsprechend behandelten Zellen auf den Phosphotyrosingehalt des PDGF-Rezeptors geprüft. Während in den DMSO-behandelten Zellen der Phosphotyrosingehalt des stimulierten PDGF-Rezeptors über die Inkubationszeit etwa gleich bleibt (Gleichgewicht von PTK- und PTPase-Reaktion) nimmt der Phosphotyrosingehalt der Rezeptoren in den mit dem PTK-Inhibitor behandelten Zellen kontinuierlich ab. Diese Abnahme wird durch die Aktivität der PDGF Rezeptor-gerichteten PTPase in den intakten Zellen bewirkt. Eine graphische Auswertung ist in Abb. 4 gezeigt.
In Kontrollexperimenten (nicht gezeigt) wurde durch Immunpräzipitation die Identität und die über den Versuchsablauf gleichbleibende Konzentration des PDGF-Rezeptors sichergestellt.

### Beispiel 2

### Messung der NGF-Rezeptor (Trk)-gerichteten PTPase(n) in PC12-Zellen mit Hilfe eines spezifischen Inhibitors der NGF-Rezeptor-PTK

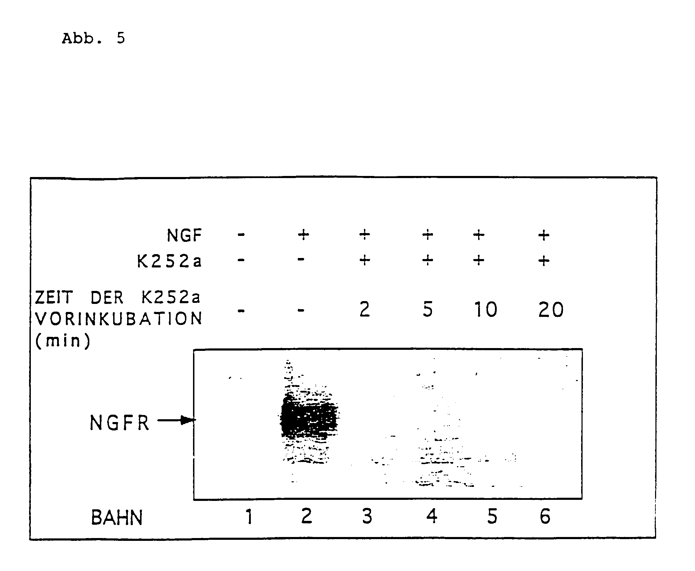

Für die dargestellten Untersuchungen wurden PC12-Zellen (Ratten Pheochromocytom-Zellen) verwendet, die NGF-Rezeptoren (Trk A) überexprimieren (Obermeier et al. (1994)). Die Stimulation der Zellen mit NGF führt zu einer raschen Autophosphorylierung dieser Rezeptoren an Tyrosinresten. Diese Autophosphorylierung ist in Immunoblots mit Anti-Phosphotyrosin-Antikörpern nach Immunpräzipitation der Rezeptoren nachweisbar. Die Identität der zellulären PTPasen, die autophosphorylierte NGF-Rezeptoren dephosphorylieren, ist nicht bekannt. Das Staurosporin-Derivat K252a wurde als spezifischer Inhibitor für die NGF-Rezeptor PTK sowie auch die PTK-Aktivität anderer Neurotrophin-Rezeptoren beschrieben (Ohmichi et al. (1992); Tapley et al. (1992)). In Analogie zu den unter Beispiel 1 beschriebenen Versuchen wurde festgestellt, daß K252a die Aktivität von Trk-Rezeptor PTK bereits nach kurzer Behandlung der Zellen vollständig hemmt (Abb. 4). Wird der Inhibitor Zellen nach Aktivierung von Trk zugesetzt, ist die sehr rasche Dephosphorylierung des NGF-Rezeptors meßbar (Abb. 5).
In Kontrollexperimenten (nicht gezeigt) wurde die über den Versuchsablauf gleichbleibende Konzentration des NGF-Rezeptors sichergestellt.

### Hemmung der NGF-Rezeptor PTK (Trk) durch den spezifischen Inhibitor K252a (Abb. 5)

TrkA überexprimierende PC12-Zellen wurden in serumfreiem Zellkulturmedium ohne Zusätze (Bahn 1,2) oder mit dem NGF-Rezeptor PTK Inhibitor K252a (Endkonzentration 1 µM) (Bahn 3-6) für verschiedene Zeiten, wie angegeben, inkubiert. Dann erfolgte die Zugabe von NGF (Endkonzentration 150 ng/ml) (Bahn 2-6) oder des entsprechenden Lösungsmittels (Bahn 1) und weitere Inkubation für 10 min. Nach Standardverfahren wurde dann ein Zellextrakt hergestellt, die NGF-Rezeptoren mit einem spezifischen Antikörper immunpräzipitiert und vergleichbare Mengen des Immunpräzipitats wurden mittels SDS-PAGE und Immunoblotting mit Anti-Phosphotyrosin-Antikörpern analysiert. Die NGF-induzierte Autophosphorylierung des Rezeptors wird bereits durch Inkubation der Zellen für 2 min mit K252a vollständig unterdrückt.

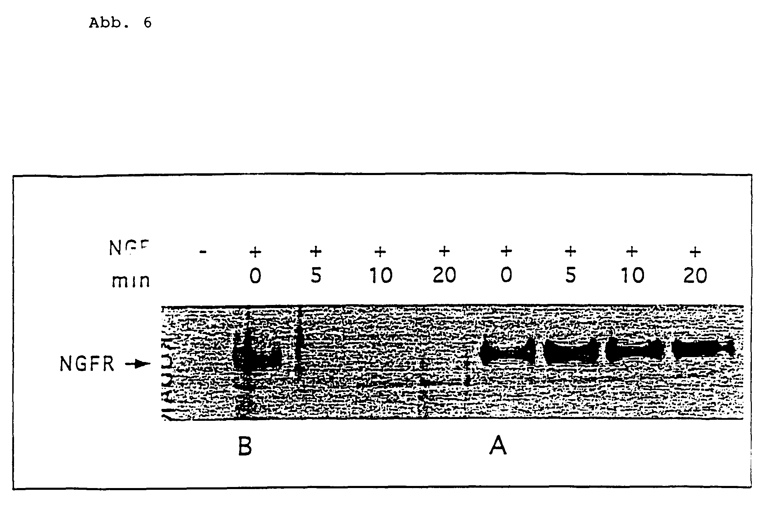

### Behandlung von intakten PC12-Zellen mit K252a erlaubt die Detektion der Aktivität NGF-Rezeptor-gerichteter PTPase(n) in vivo (Abb. 6)

TrkA transfizierte PC12-Zellen wurden in serumfreiem Medium mit NGF (Bahn 2-6) oder dem entsprechenden Lösungsmittel (Bahn 1) behandelt. Nach 10-minütiger Stimulation wurde K252a (1 µM, B) oder entsprechendes Lösungsmittel (DMSO, A) zugesetzt. Zu verschiedenen Zeitpunkten, wie angegeben, wurden die entsprechend behandelten Zellen wie in Abb. 1 beschrieben aufgearbeitet und die NGF-Rezeptoren auf ihren Phosphotyrosingehalt geprüft. Während in den DMSO-behandelten Zellen der Phosphotyrosingehalt des stimulierten NGF-Rezeptors über die Inkubationszeit etwa gleich bleibt (Gleichgewicht von PTK- und PTPase-Reaktion) nimmt der Phosphotyrosingehalt des Rezeptors in den mit dem PTK-Inhibitor behandelten Zellen drastisch ab. Diese Abnahme wird durch die Aktivität der NGF-Rezeptorgerichteten PTPase in den intakten Zellen bewirkt.

### Beispiel 3

### Der erfindungsgemäße Test für die PDGF-Rezeptor-gerichtete(n) PTPase(n) gestattet den Nachweis von PTPase-Effektoren

Vorbehandlung von Swiss 3T3-Zellen mit potentiellen Effektoren der für den PDGF-Rezeptor spezifischen PTPasen beschleunigt (PTPase-Aktivatoren) oder verlangsamt (PTPase-Inhibitoren) die Dephosphorylierung. In Tabelle 1 ist der in dieser Weise gemessene Effekt von den bekannten, unspezifischen PTPase-Inhibitoren Na-Orthovanadat und Phenylarsinoxid sowie des erfindungsgemäßen Aktivators A23187 gezeigt.

Swiss 3T3-Zellen wurden mit den in Tabelle 1 aufgeführten Effektoren (oder entsprechenden Lösungsmitteln) behandelt. Dann wurde die Aktivität der PDGF-Rezeptor-PTPase(n) mit dem vorstehend beschriebenen Test analysiert. Die Immunoblots wurden durch direkte Fluoreszenzdetektion (Imaging System G250, Biorad) oder durch Filmexposition und Densitometrie (Image 1.04) quantifiziert. Die relative Dephosphorylierung des PDGF-Rezeptors nach 5 min wurde in % im Vergleich zu Kontrollexperimenten ohne Effektor (100 %) angegeben.

**Tabelle 1**

| Effektor | Relative PDGF Rezeptor-gerichtete PTPase-Aktivität (% der Kontrolle) |
|---|---|
| Na-Orthovanadat (2 mM) | 34 |
| | |
| Phenylarsinoxid (PAO) (25 µM) | 38 |
| | |
| A 23187 (1 µM) | 161 |

### Literatur

Barinaga, M. (1994), Science 264, 772-774.

Böhmer, F.D., Böhmer, S.A. und Heldin, C.H. (1993), FEBS Lett. 331, 276-280.

Burke, T.R. (1992), Frugs of the Future 17, 119-131.

Charbonneau, H. und Tonks, N.K. (1992), Annu. Rev. Cell Biol. 8, 463-493.

Debono, M., Molloy, R.M., Dorman, D.E., Paschal, J.W., Babcock, D.F., Deber, C.M. und Pfeiffer, D.R. (1981), Biochemistry 20, 6865-6872.

Faure, R., Baquiran, G., Bergeron, J.J.M. und Posner, B.I. (1992), J. Biol. Chem. 267, 11215-11221.

Goldstein, B. (1992), J Cell Biochem 48, 33-42.

Heldin, C.H. und Westermark, B. (1991), CRC Crit. Rev. Oncogenesis 2, 109-124.

Hosang, M., Rouge, M., Wipf, B., Eggimann, B., Kaufmann, F. und Hunziker, W. (1989), J. Cell. Physiol. 140, 558-564.

Levitzky, A. (1992), FASEB J. 6, 3275-3282.

Obermeier, A., Bradshaw, R.A., Seedorf, K., Choidas, A., Schlessinger, J. und Ullrich, A. (1994), EMBO J 13, 1585-1590.

Ohmichi, M., Decker, S., Pang, L. und Saltiel, A. (1992), Biochemistry 31, 4034-4039.

Tapley, P., Lamballe, F. und Barbacid, M. (1992), Oncogene 7, 371-381.

Mooney, R.A. und Bordwell, K.L. (1992), J. Biol. Chem. 267, 14054-14060.

Pot, D.A. und Dixon, J.E. (1992), Biochim. Biophys. Acta 1136, 35-43.

Ross, R. (1993), Nature 362, 801-809.

Rubin, K., Terracio, L., Rönnstrand, L., Heldin, C.H. und Klareskog, L. (1988), Scand. J. Immunol. 27, 285-294.

Shaw, R.J., Benedict, S.H., Clark, R.A. und King, T.E. (1991), Am. Rev. Resp. Dis. 143, 167-173.

Snider, W.D. (1994), Cell 77, 627-638.

Swarup, G., Cohen, S. und Garbers, D.L. (1982), Biochem.Biophys.Res.Comm. 107, 1104-1109.

Ullrich, A. und Schlessinger, J. (1990), Cell 61, 203-212.

## Patentansprüche

1. Verfahren zur Bestimmung der Aktivität einer spezifischen Proteintyrosinphosphatase (PTPase) in intakten Zellen,
**dadurch gekennzeichnet**,
daß man
(a) intakte Zellen, in denen ein Substrat der zu bestimmenden PTPase enthalten ist, gegebenenfalls nach Stimulierung der Substratphosphorylierung, mit mindestens einer Substanz, welche in die intakte Zelle eindringen und selektiv die Phosphorylierung des Substrats inhibieren kann, in einer wirksamen Konzentration inkubiert,
(b) nach einer gegebenen Inkubationsdauer den Phosphorylierungsgrad des Substrats mißt,
(c) gegebenenfalls Schritt (b) mindestens einmal wiederholt und
(d) aus dem gemessenen Phosphorylierungsgrad des Substrats die Aktivität der spezifischen PTPase bestimmt.

2. Verfahren nach Anspruch 1,
**dadurch gekennzeichnet**,
daß die Substanz ein selektiver Inhibitor einer das Substrat phosphorylierenden Proteintyrosinkinase (PTK) ist.

3. Verfahren nach einem der vorhergehenden Ansprüche,
**dadurch gekennzeichnet**,
daß die spezifische Proteintyrosinkinase eine Untereinheit des Substrats ist, so daß die Phosphorylierung eine Autophosphorylierung ist.

4. Verfahren nach einem der vorhergehenden Ansprüche,
**dadurch gekennzeichnet**,
daß man als Substrat einen Zelloberflächenrezeptor verwendet.

5. Verfahren nach Anspruch 4,
**dadurch gekennzeichnet**,
daß man als Substrat einen Rezeptor für einen Wachstumsfaktor verwendet.

6. Verfahren nach Anspruch 5,
**dadurch gekennzeichnet**,
daß der Rezeptor ausgewählt wird aus NGF-Rezeptor, PDGF-Rezeptor, KIT/SCF-Rezeptor, KD-Rezeptor, EGF-Rezeptor und Insulin-Rezeptor.

7. Verfahren nach Anspruch 6,
**dadurch gekennzeichnet**,
daß man als Substrat einen PDGF-Rezeptor und als PTK-Inhibitor AG 1295 und/oder AG 1296 verwendet.

8. Verfahren nach Anspruch 6,
**dadurch gekennzeichnet**,
daß man als Substrat einen NGF-Rezeptor und als PTK-Inhibitor K252 A verwendet.

9. Verfahren nach Anspruch 1 zur Identifizierung von Effektoren einer spezifischen Proteintyrosinphosphatase,
**dadurch gekennzeichnet**,
daß man eine zusätzliche Bestimmung in Gegenwart einer Testsubstanz durchführt und aus dem Vergleich der Phosphorylierungsgrade die Wirkung der Testsubstanz als Effektor der PTPase ermittelt.

## Claims

1. Method for the determination of the activity of a specific protein tyrosine phosphatase (PTPase) in intact cells,
**wherein**,
(a) intact cells which contain a substrate of the PTPase to be determined are incubated, if desired after stimulating substrate phosphorylation, with an effective concentration of at least one substance which can penetrate into the intact cell and can selectively inhibit the phosphorylation of the substrate,
(b) the degree of phosphorylation of the substrate is measured after a given incubation period,
(c) if desired, step (b) is repeated at least once and
(d) the activity of the specific PTPase is determined from the measured degree of phosphorylation of the substrate.

2. Method as claimed in claim 1,
**wherein**
the substance is a selective inhibitor of a protein tyrosine kinase (PTK) which phosphorylates the substrate.

3. Method as claimed in one of the previous claims,
**wherein**
the specific protein tyrosine kinase is a subunit of the substrate so that the phosphorylation is an autophosphorylation.

4. Method as claimed in one of the previous claims,
**wherein**
a cell surface receptor is used as the substrate.

5. Method as claimed in claim 4,
**wherein**
a receptor for a growth factor is used as the substrate.

6. Method as claimed in claim 5,
**wherein**
the receptor is selected from NGF receptor, PDGF receptor, KIT/SCF receptor, KD receptor, EGF receptor and insulin receptor.

7. Method as claimed in claim 6,
**wherein**
a PDGF receptor is used as the substrate and AG 1295 and/or AG 1296 is used as a PTK inhibitor.

8. Method as claimed in claim 6,
**wherein**
a NGF receptor is used as the substrate and K252 A is used as a PTK inhibitor.

9. Method as claimed in claim 1 to identify effectors of a specific protein tyrosine phosphatase,
**wherein**
an additional determination is carried out in the presence of a test substance and the action of the test substance as an effector of PTPase is determined from the comparison of the degrees of phosphorylation.

## Revendications

1. Procédé pour la détermination de l'activité d'une protidotyrosine phosphatase spécifique (PTPase) dans des cellules intactes, caractérisé en ce que,
(a) l'on met en incubation dans une concentration efficace, des cellules intactes dans lesquelles est contenu un substrat de la PTPase à déterminer, éventuellement après stimulation de la phosphorylation de substrat, avec au moins une substance qui peut pénétrer dans les cellules intactes et inhiber sélectivement la phosphorylation du substrat,
(b) après une durée d'incubation donnée, on mesure le degré de phosphorylation du substrat,
(c) on répète éventuellement l'étape (b) au moins une fois
(d) à partir du degré de phosphorylation mesuré du substrat, on détermine l'activité de la PTPase spécifique.

2. Procédé selon la revendication 1, caractérisé en ce que la substance est un inhibiteur sélectif d'une protidotyrosinekinase (PTK) phosphorylant le substrat.

3. Procédé selon l'une des revendications précédentes, caractérisé en ce que la protidotyrosinekinase spécifique est une sous-unité du substrat de sorte que la phosphorylation est une autophosphorylation.

4. Procédé selon l'une des revendications précédentes, caractérisé en ce que,
l'on utilise comme substrat un récepteur superficiel de cellules.

5. Procédé selon la revendication 4, caractérisé en ce que comme substrat on utilise un récepteur pour un facteur de croissance.

6. Procédé selon la revendication 5, caractérisé en ce que le récepteur est choisi à partir de récepteur NGF, récepteur PDGF, récepteur KIT/SCF, récepteur KD, récepteur EGF et récepteur insuline.

7. Procédé selon la revendication 6, caractérisé en ce qu'en tant que substrat, on utilise un récepteur PDGF et en tant qu'inhibiteur PTK AG 1295 et/ou AG 1296.

8. Procédé selon la revendication 6, caractérisé en ce qu'en tant que substrat, on utilise un récepteur NGF et en tant qu'inhibiteur PTK R 252 A.

9. Procédé selon la revendication 1, pour l'identification d'effecteurs d'une protidotyrosine phosphatase spécifique,
caractérisé en ce que,
l'on procède à une détermination supplémentaire en présence d'une substance test et à partir de la comparaison des degrés de phosphorylation, on calcule l'effet de la substance test en tant qu'effecteur de la PTPase.
